# FASCICULE DE BREVET EUROPEEN

(11) **EP 2 567 752 B1**
(45) Date de publication et mention de la délivrance du brevet: **21.11.2018**
(21) Numéro de dépôt: 12290277.8
(22) Date de dépôt: 20.08.2012
(51) Int. Cl.: B01J 31/18, C07C 2/32, C07C 2/34

(54) **NOUVELLE COMPOSITION CATALYTIQUE À BASE DE NICKEL ET PROCÉDÉ D'OLIGOMÉRISATION DES OLÉFINES UTILISANT LADITE COMPOSITION**
NEUARTIGE KATALYTISCHE ZUSAMMENSETZUNG AUF NICKELBASIS, UND OLIGOMERISATIONSVERFAHREN VON OLEFINEN, BEI DER DIESE ZUSAMMENSETZUNG ZUM EINSATZ KOMMT
NEW NICKEL-BASED CATALYTIC COMPOSITION AND METHOD FOR OLIGOMERISING OLEFINS USING SAID COMPOSITION

(30) Priorité: 08.09.2011 FR 1102731
(43) Date de publication de la demande: 13.03.2013
(73) Titulaire: IFP Energies nouvelles, 92500 Rueil-Malmaison (FR)
(72) Inventeur: Breuil, Pierre-Alain, 69006 Lyon (FR); Boudier, Adrien, 69008 Lyon (FR); Magna, Lionel, 69007 Lyon (FR); Olivier-Bourbigou, Helene, 69230 Saint Genis-Laval (FR)
(74) Mandataire: IFP Energies nouvelles

(56) Documents cités:
- WO-A1-02/38625
- US-A1- 2006 094 588
- DATABASE CA [Online] CHEMICAL ABSTRACTS SERVICE, COLUMBUS, OHIO, US; 2010, WU, AIZHI ET AL: "Antimicrobial activities and synthesis of four new complexes with Schiff base condensed by 1-methyl-2-imidazolecarboxaldehyde and 2-aminoethanol", XP002671896, extrait de STN Database accession no. 2010:1425762 & WU, AIZHI ET AL: "Antimicrobial activities and synthesis of four new complexes with Schiff base condensed by 1-methyl-2-imidazolecarboxaldehyde and 2-aminoethanol", GUANGZHOU HUAXUE , 35(2), 27-30 CODEN: GAHUFW; ISSN: 1009-220X, 2010,
- DATABASE CA [Online] CHEMICAL ABSTRACTS SERVICE, COLUMBUS, OHIO, US; 2000, POPESCU, ANGELA ET AL: "Carbonic anhydrase inhibitors. Part 67. Schiff bases of some aromatic sulfonamides and their metal complexes: towards more selective inhibitors of carbonic anhydrase isozyme IV", XP002671897, extrait de STN Database accession no. 2000:836053 & POPESCU, ANGELA ET AL: "Carbonic anhydrase inhibitors. Part 67. Schiff bases of some aromatic sulfonamides and their metal complexes: towards more selective inhibitors of carbonic anhydrase isozyme IV", JOURNAL OF ENZYME INHIBITION , 14(6), 407-423 CODEN: ENINEG; ISSN: 8755-5093, 1999,
- HONGSHAN HE ET AL: "Structural and Spectroscopic Study of Reactions between Chelating Zinc-Binding Groups and Mimics of the Matrix Metalloproteinase and Disintegrin Metalloprotease Catalytic Sites: The Coordination Chemistry of Metalloprotease Inhibition", INORGANIC CHEMISTRY, vol. 44, no. 21, 1 octobre 2005 (2005-10-01), pages 7431-7442, XP055022404, ISSN: 0020-1669, DOI: 10.1021/ic050723p

## Description

La présente invention concerne l'oligomérisation des oléfines comportant de 2 à 10 atomes de carbone et en particulier la dimérisation de l'éthylène. Un objet de l'invention est de fournir une nouvelle composition catalytique à base de nickel et un procédé d'oligomérisation des oléfines utilisant ladite composition catalytique particulière.

### Art antérieur:

Il est connu de préparer des compostions catalytique de dimérisation ou de codimérisation de monooléfines telles que l'éthylène, le propylène, les butènes ou les pentènes. Parmi ces catalyseurs, on peut citer notamment à titre d'exemples : les produits obtenus par réaction des halogénures de π-allyl nickel phosphine avec les acides de Lewis, comme cela est décrit dans le brevet français FR-B-1 410 430, les produits obtenus par réaction des halogénures de nickel phosphine avec les acides de Lewis, comme cela est décrit dans le brevet US-A-3 485 881 et les produits obtenus par réaction de certains carboxylates de nickel avec les halogénures d'hydrocarbylaluminium, comme cela est décrit dans le brevet US-A-3 321 546.

Presque toutes ces compositions catalytiques mettent en oeuvre un ligand qui est un composé organique du phosphore. Ces systèmes sont connus pour être sélectifs pour la dimérisation de l'éthylène comme cela est décrit dans le brevet US 3 485 881. Cependant, il est préférable de pouvoir disposer de catalyseurs d'oligomérisation sans phosphore. Une possibilité serait d'utiliser des catalyseurs dans lesquels le nickel se trouve déposé sur un support minéral comportant des sites acides, comme la silice, l'alumine ou les silice-alumines. Cependant, il s'agit là de catalyseurs solides, contrairement aux compositions catalytiques en phase liquide de l'invention.

Il a été décrit récemment, dans les demandes de brevet WO 2004/083263 et WO 2005/111099, l'utilisation de compositions catalytiques comportant un précurseur de nickel, de chrome ou de vanadium et au moins un ligand de type imino-imidazole, fonctionnalisé ou non, et de préférence de type imino-benzimidazole fonctionnalisé, permettant l'oligomérisation et la (co)polymérisation de l'éthylène avec, ou non, une autre oléfine.

Un objectif de l'invention est de fournir une nouvelle composition catalytique pour l'oligomérisation des oléfines comportant de 2 à 10 atomes de carbone et en particulier la dimérisation de l'éthylène.

Un autre objectif de l'invention est de fournir un procédé d'oligomérisation des oléfines comportant de 2 à 10 atomes de carbone et en particulier un procédé de dimérisation de l'éthylène, mettant en oeuvre ladite composition catalytique, ledit procédé présentant une activité catalytique améliorée.

II a maintenant été trouvé, de façon inattendue, qu'une nouvelle composition imino-imidazole fonctionnalisé ou non, en combinaison avec éventuellement un agent activateur, en présence d'un solvant ou pas, présente une activité améliorée pour l'oligomérisation des oléfines comportant de 2 à 10 atomes de carbone, en particulier la dimérisation des oléfines, et encore plus particulièrement une sélectivité très élevée pour la dimérisation de l'éthylène.

### Description détaillée de l'invention:

Un premier objet de l'invention concerne une nouvelle composition catalytique comprenant au moins un complexe de nickel, ledit complexe étant obtenu à partir d'un mélange comprenant :
- au moins un précurseur de nickel A, avec
- au moins un ligand imino-imidazole B de formule (I) 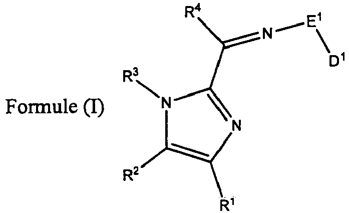 tel que définie dans les revendications annexées.

De préférence, R¹, R², R³ et R⁴ sont choisis parmi les groupements hydrogène, méthyle, éthyle, isopropyle, *iso*-butyle, *tert*-butyle, cyclohexyle, phényle et benzyle; De manière préférée, R¹, R² et R⁴ sont des atomes d'hydrogène, et de manière préférée, R³ est un groupement méthyle.

E¹ est un groupement aliphatique ou aromatique. De manière préférée, E¹ est choisi parmi les groupes -CH₂-, -CH₂CH₂-, -CH₂CH₂CH₂-, -CH₂CH₂CH₂CH₂-, 1,2-phenylène, *trans*-1,2-cyclopentane, *trans*-1,2-cyclohexane, 2,3-butane, 1,1'-biphényle, CH₂CH₂- ou le groupement divalent 1,2-phenylène.

D¹ est choisi parmi un atome d'hydrogène, un éther de formule -OR⁵, un thioéther de formule -SR⁶, une amine de formule -N(R⁷)₂ ou une phosphine de formule - P(R⁸)₂ où R⁵, R⁶, R⁷ et R⁸ sont des groupements méthyles, éthyles ou phényle.
La préparation desdits ligands imino-imidazole B de formule (I) s'effectue selon les méthodes connues de la littérature. Par exemple, lesdits ligands peuvent être préparés selon la méthode de préparation décrite dans les publications Org. Lett. 2007, 9, 18, 3699-3701 ou Inorg. Chem. 2005, 44, 7431-7442.
Les exemples donnés dans la présente invention ne sont donnés qu'à titre indicatif et ne limitent pas la présente invention.

Ledit précurseur de nickel A utilisé dans la composition catalytique selon l'invention est avantageusement choisi parmi le chlorure de nickel(II), le chlorure de nickel(II)(dimétoxyéthane), le bromure de nickel(II), le bromure de nickel(II)(dimétoxyéthane), le fluorure de nickel(II), l'iodure de nickel(II), le sulfate de nickel(II), le carbonate de nickel(II), le dimethylglyoxime de nickel(II), l'hydroxyde de nickel(II), l'hydroxyacétate de nickel(II), l'oxalate de nickel(II), les carboxylates de nickel(II) tel que par exemple le 2-éthylhexanoate, les phénates de nickel, l'acétate de nickel(II), le trifluoroacétate de nickel(II), le triflate de nickel(II), l'acétylacétonate de nickel(II), l'hexafluoroacétylacétonate de nickel(II), le nickel bis(cycloocta-1,5-diène), le nickel bis(cycloocta-1,3-diène), le nickel bis(cyclooctatétraène), le nickel bis(cycloocta-1,3,7-triène), le bis(o-tolylphosphito)nickel(éthylène), le nickel tétrakis (triphénylphosphite), le nickel bis(éthylène), le chlorure de π-allylnickel(II), le bromure de π-allylnickel(II), le dimère du chlorure de methallylnickel(II), l'hexafluorophosphate de η³-allylnickel(II), l'hexafluorophosphate de η³-methallylnickel(II) et le 1,5-cyclooctadiényle de nickel(II), sous leur forme hydraté ou non, pris seul ou en mélange. Lesdits précurseurs de nickel peuvent éventuellement être complexés à des bases de Lewis.
Lesdits précurseurs de nickel utilisés dans la composition catalytique selon l'invention sont avantageusement préparés selon les méthodes connues de la littérature. Par exemple, lesdits précurseurs de nickel peuvent être préparés selon la méthode de préparation décrite dans les demandes de brevets WO 2004/083963 ou WO 2001/74831 ou dans la publication New J. Chem. 2011, 35, 178-183.

La composition catalytique selon l'invention peut avantageusement également contenir un composé C appelé agent activateur.

Ledit agent activateur est avantageusement choisi dans le groupe formé par les composés tris(hydrocarbyl)aluminium, les composés chlorés ou bromés d'hydrocarbylaluminium, les aluminoxanes, les composés organiques susceptibles de donner un proton, les composés organo-borés, pris seuls ou en mélange.
Les tris(hydrocarbyl)aluminium et les composés chlorés ou bromés d'hydrocarbylaluminium répondent de préférence à la formule générale AlₓR"_{y}W_{z} dans laquelle R" représente un radical hydrocarboné monovalent contenant par exemple jusqu'à 12 atomes de carbone tel que alkyle, aryle, aralkyle, alkaryle ou cycloalkyle, W représente un atome d'halogène choisi par exemple parmi le chlore et le brome, W étant de préférence un atome de chlore, x prend une valeur de 1 à 2, y et z prennent une valeur de 1 à 3. Comme exemples de tels composés, on peut mentionner le sesquichlorure d'éthylaluminium (Et₃Al₂Cl₃), le dichlorure de méthylaluminium (MeAlCl₂), le dichlorure d'éthylaluminium (EtAlCl₂), le dichlorure d'isobutylaluminium (iBuAlCl₂), le chlorure de diéthylaluminium (Et₂AlCl), le triméthylaluminium, le tributylaluminium, le tri*-n*-octylaluminium et le triéthylaluminium (AlEt₃).
Dans le cas ou ledit agent activateur est choisi parmi les aluminoxanes, ledit agent activateur est avantageusement choisi parmi le méthylaluminoxane (MAO), l'éthylaluminoxane et le méthylaluminoxane modifié (MMAO). Ces agents activateurs peuvent être utilisé seuls ou en mélange.
De préférence, ledit agent activateur C est choisi parmi le dichloroéthylaluminium (EtAlCl₂) et le méthylaluminoxane (MAO).
Dans le cas ou ledit agent activateur est choisi parmi les composés organiques susceptibles de donner un proton, ledit agent activateur est de préférence, choisis parmi les acides de formule HX dans lequel X représente un anion.
Dans le cas ou ledit agent activateur est choisi parmi les composés organoborés, ledit agent activateur est de préférence choisi parmi les acides de Lewis de type tris(aryl)borane tels que le tris(perfluorophényl)borane, le tris(3,5-bis(trifluorométhyl)phényl)borane, le tris(2,3,4,6-tétrafluorophényl)borane, le tris(perfluoronaphtyl)borane, le tris(perfluobiphényl)borane et leurs dérivés et les (aryl)borates associé à un cation triphénylcarbénium ou à un cation ammonium trisubstitué tels que le triphénylcarbenium tétrakis(perfluorophényl)borate, le N,N-diméthylanilinium tétrakis(perfluorophényl)borate, le N,N-diéthylanilinium tétrakis(3,5-bis(trifluorométhyl)phényl)borate, le triphénylcarbenium tétrakis(3,5-bis(trifluorométhyl)phényl)borate.

Un autre objet de la présente invention concerne un procédé d'oligomérisation des oléfines comportant de 2 à 10 atomes de carbone utilisant ladite composition catalytique. De préférence, ledit procédé est un procédé d'oligomérisation de l'éthylène, de manière préférée un procédé de dimérisation de l'éthylène.

Ledit précurseur de nickel A et ledit ligand imino-imidazole B de formule (I) peuvent être avantageusement mélangés pour obtenir ledit complexe de nickel en présence d'un solvant ou pas, appelé solvant de préparation. Ledit complexe de nickel de la composition catalytique selon l'invention est avantageusement préparé selon tout procédé de préparation connu de l'homme du métier.
Ledit solvant de préparation peut avantageusement être identique ou différent du solvant de réaction, c'est-à-dire du solvant utilisé pour le procédé d'oligomérisation selon l'invention et de préférence pour le procédé de dimérisation. Lesdits solvants de préparation et de réaction sont avantageusement choisis parmi les solvants organiques et de préférence parmi les éthers, les alcools, les solvants chlorés et les hydrocarbures saturées, insaturés, aromatiques ou non, cycliques ou non. De préférence, lesdits solvants sont choisis parmi l'hexane, le cyclohexane, l'heptane, le butane ou l'isobutane, les monooléfines ou dioléfines comportant de préférence 4 à 20 atomes de carbone, le benzène, le toluène, l'ortho-xylène, le mésitylène, l'éthylbenzène, le dichlorométhane, le chlorobenzène, le méthanol, l'éthanol, purs ou en mélange, le mélange d'oléfines produit par ledit procédé d'oligomérisation et les liquides ioniques. Dans le cas où lesdits solvants sont un liquide ionique, ils sont avantageusement choisis parmi les liquides ioniques décrits dans les brevets US 6,951,831 B2 et FR 2895406 B1.
Ledit complexe de nickel de la composition catalytique dilué ou non dans ledit solvant de préparation peut avantageusement être utilisé dans le procédé d'oligomérisation selon l'invention et de préférence dans le procédé de dimérisation. Un agent activateur C peut ensuite, éventuellement être ajouté.
Ledit complexe de nickel de la composition catalytique peut également avantageusement être isolé puis utilisé, dilué ou non dans un solvant, dans le procédé d'oligomerisation selon l'invention et de préférence dans le procédé de dimérisation. Un agent activateur C peut ensuite, éventuellement être ajouté.
Ladite composition catalytique selon l'invention peut également avantageusement être préparée *in situ* dans la section réactionnelle et dans le solvant utilisé pour le procédé d'oligomérisation selon l'invention et de préférence pour le procédé de dimérisation. Dans ce cas, l'ordre de mélange du précurseur de nickel A, du ligand imino-imidazole B de formule (I) et éventuellement de l'agent activateur C, n'est pas critique.

L'oligomérisation est définie comme la transformation d'une unité monomère en un composé ou mélange de composés de formule générale CpH2p avec 4 ≤ p ≤ 80, de préférence avec 4 ≤ p ≤ 50, de manière préférée avec 4 ≤ p ≤ 26 et de manière plus préférée avec 4 ≤ p ≤ 14.

Les oléfines utilisées dans le procédé d'oligomérisation selon l'invention sont des oléfines comportant de 2 à 10 atomes de carbone et de préférence lesdites oléfines sont choisies parmi l'éthylène, le propylène, les n-butènes et les n-pentènes, seules ou en mélange, pures ou diluées. Dans le cas où lesdites oléfines sont diluées, lesdites oléfines sont diluées par un ou plusieurs alcane(s), tels qu'on les trouve dans des « coupes » issues des procédés de raffinage du pétrole, comme le craquage catalytique ou le craquage à la vapeur.
De manière très préférée, l'oléfine utilisée dans le procédé d'oligomérisation selon l'invention est de l'éthylène.
Lesdites oléfines peuvent venir de ressources non fossiles telles que la biomasse. Par exemple, les oléfines utilisées dans le procédé d'oligomérisation selon l'invention peuvent avantageusement être produites à partir d'alcools, et en particulier par déshydratation des alcools. Dans un mode de réalisation préféré, lesdites oléfines sont produites par déshydratation de l'éthanol pour produire de l'éthylène.

La concentration du nickel dans la solution catalytique est avantageusement comprise entre 1.10⁻⁵ et 1 mol/L, et de préférence entre 5.10⁻⁵ et 1.10⁻² mol/L.
Le rapport molaire entre le ligand B de formule (I) et le précurseur de nickel A est avantageusement compris entre 0,05 et 10, de préférence entre 0,5 et 2 et préférentiellement 1.
Le rapport molaire entre l'agent activateur C et le complexe de nickel est avantageusement compris entre 1/1 et 1000/1, de préférence entre 100/1 et 1000/1 pour les aluminoxanes et de préférence entre 1/1 et 20/1 pour les autres dérivés d'aluminium et les autres acides de Lewis.

Le procédé d'oligomérisation selon l'invention et de préférence le procédé de dimérisation de l'éthylène opère avantageusement à une pression totale comprise entre la pression atmosphérique et 20 MPa, de préférence entre 0,5 et 8 MPa, et à une température comprise entre -40 et +250°C, de préférence entre -20°C et 150°C.
La chaleur engendrée par la réaction peut avantageusement être éliminée par tous les moyens connus de l'homme du métier.
Le procédé d'oligomérisation selon l'invention et de préférence le procédé de dimérisation de l'éthylène peut avantageusement être conduit en système fermé, en système semi-ouvert ou en continu, avec un ou plusieurs étages de réaction. Une vigoureuse agitation est avantageusement mise en oeuvre pour assurer un bon contact entre le ou les réactifs et la composition catalytique.
Le procédé d'oligomérisation selon l'invention et de préférence le procédé de dimérisation de l'éthylène peut avantageusement être mis en oeuvre en discontinu. Dans ce cas, un volume choisi de la solution comprenant la composition catalytique selon l'invention est introduit dans un réacteur muni des dispositifs habituels d'agitation, de chauffage et de refroidissement. La pression d'éthylène et la température sont ajustées aux valeurs souhaitées. La pression au sein du réacteur est maintenue constante par introduction d'éthylène jusqu'a ce que le volume total de liquide produit représente, par exemple, 2 à 50 fois le volume de la solution comprenant la composition catalytique primitivement introduit. La composition catalytique est détruite par tout moyen habituel connu de l'homme du métier, puis les produits sont soutirés et séparés du solvant.
Le procédé d'oligomérisation selon l'invention et de préférence le procédé de dimérisation de l'éthylène peut également avantageusement être mis en oeuvre en continu. Dans ce cas, la solution comprenant la composition catalytique selon l'invention est injectée en même temps que l'éthylène dans un réacteur agité par les moyens mécaniques classiques ou par une recirculation extérieure, et maintenue à la température souhaitée. Dans un autre mode de réalisation, les composants de ladite composition catalytique peuvent aussi être injectés de manière séparée, par exemple le précurseur de nickel A et le ligand B d'une part et l'agent activateur C d'autre part. L'éthylène est introduit par une vanne d'admission asservie à la pression, qui maintient celle-ci constante. Le mélange réactionnel est soutiré au moyen d'une vanne asservie au niveau liquide de façon à maintenir celui-ci constant. La composition catalytique est détruite en continu par tout moyen habituel connu par l'homme du métier, puis les produits de la réaction ainsi que le solvant sont séparés, par exemple par distillation. L'oléfine qui n'a pas été transformée peut être recyclée dans le réacteur.
Ledit procédé selon l'invention peut avantageusement être mis en oeuvre dans un réacteur à un ou plusieurs étages de réaction en série, la charge oléfinique et/ou la composition catalytique au préalable pré-conditionnée étant introduites en continu, soit dans le premier étage, soit dans le premier et un autre quelconque des étages. A la sortie du réacteur, la composition catalytique peut être désactivée, par exemple par injection d'ammoniac et/ou d'une solution aqueuse de soude et/ou d'une solution aqueuse d'acide sulfurique. Les oléfines non converties et les alcanes éventuellement présents dans la charge sont ensuite séparés des oligomères par distillation.

Les produits du présent procédé peuvent trouver une application par exemple comme composants de carburants pour automobiles, comme charges dans un procédé d'hydroformylation pour la synthèse d'aldéhydes et d'alcools et/ou comme charges dans un procédé de métathèse pour la synthèse de propylène par exemple.

Les exemples suivants illustrent l'invention sans en limiter la portée.

### Exemples selon l'invention

### Exemple 1

### Préparation du ligand L-1 (composé B)

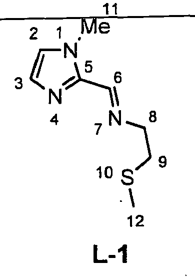

A une solution de 1-méthyle-2-imidazolecarboxyaldéhyde (490 mg ; 4,45 mmol) dans 15 mL de dichlorométhane, est ajoutée la 2-(methylthio)ethylamine (446,1 mg ; 4.89 mmol). La solution de couleur jaune est agitée pendant 12 heures à température ambiante. Le solvant est ensuite évaporé. Le ligand L-1 est obtenu sous forme d'une huile jaune pâle avec un rendement de 90 %. Les caractérisations RMN ¹H, ¹³C et IR confirment la structure du ligand L-1.
¹H NMR (300 MHz, CD₂Cl₂) : δ = 8.28 (dd, *J* = 1.9, 1.3 Hz, 1H, *H₆*), 7.05 (d, *J* = 1.0 Hz, 1H, *H₃*), 6.95 (s, 1H, *H₂*), 3.95 (s, 3H, *H₁₁*), 3.77 (td, *J* = 6.8, 1.3 Hz, 2H, *H₈*), 2.79 (t, *J* = 6.8 Hz, 2H, *H₉*), 2.12 (s, 3H, H₁₂).
¹³C NMR (75 MHz, CD₂Cl₂) : δ = 154.57 (1C, *C₆*), 143.45 (1C, *C₅*), 129.36 (1C, *C₃*), 125.22 (1C, *C₂*), 61.33 (1C, *C₈*), 35.48 (2C, *C₉*, *C₁₁*), 15.83 (1C, *C₁₂*).
FT-IR : 2914w, 1648s, 1517w, 1476m, 1435s, 1366w, 1286m, 1228w, 1147w, 1044w, 919w, 793w, 756m, 707m, 689m.

### Exemple 2

### Préparation du complexe Ni-1

A une suspension de précurseur de nickel (composé A) NiCl₂,diméthoxyéthane dans 10 mL de dichlorométhane (325 mg ; 1,48 mmol), est ajoutée goutte-à-goutte une solution jaune de ligand L-1 (284 mg ; 1,55 mmol) dans 15 mL de dichlorométhane. Le milieu réactionnel vire instantanément au vert. La suspension obtenue est agitée pendant 12 heures à température ambiante. Le précipité vert pâle formé est filtré via une canule, lavé à froid avec du dichlorométhane (3 × 20 mL), puis séché sous vide. On obtient ainsi le complexe Ni-1. Le rendement de la réaction est de 95 %.

### Exemple 3

### Préparation du ligand L-2

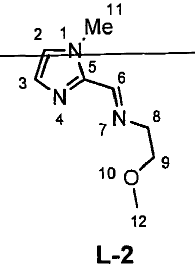

Pour la synthèse du ligand, on opère comme dans l'Exemple 1 à ceci près que l'amine utilisée est la 2-(méthoxy)éthylamine. Le ligand L-2 est obtenu sous forme d'une huile jaune pâle avec un rendement de 92 %. Les caractérisations RMN ¹H, ¹³C et IR confirment la structure du ligand L-2.
¹H NMR (300 MHz, CD₂Cl₂) : δ = 8.26 (s, 1H, *H₆),* 7.04 (s, 1H, *H₃*), 6.94 (s, 1H, *H₂*), 3.95 (s, 3H, *H₁₁*), 3.75-3.69 (m, 2H, *H₉*), 3.66-3.59 (m, 2H, *H₈*), 3.33 (s, 3H, *H₁₂*).
¹³C NMR (75 MHz, CD₂Cl₂) : δ = 154.96 (*C₆*), 143.59 (*C₅*), 129.32 (*C₃*), 125.12 (*C₂*), 72.53 (*C₉*), 61.64 (*C₈*), 58.86 (*C₁₂*), 35.46 (*C₁₁*).
FT-IR : 2875w, 1650s, 1516w, 1475m, 1437s, 1366w, 1287m, 1191w, 1118s, 1053w, 1026w, 955w, 919w, 832w, 802m, 757m, 708m, 690m.

### Exemple 4

### Préparation du complexe Ni-2

Pour la synthèse du complexe, on opère comme dans l'Exemple 2 à ceci près que le ligand utiliser pour complexer le nickel est le ligand L-2. Le complexe Ni-2 est obtenu sous forme d'un solide bleu pâle avec un rendement de 94 %.

### Exemple 5

### Préparation du ligand L-3

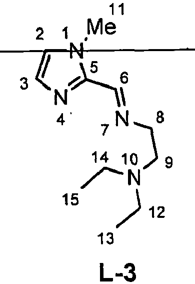

Pour la synthèse du ligand, on opère comme dans l'Exemple 1 à ceci près que l'amine utilisée est la N,N-diéthyléthylènediamine. Le ligand L-3 est obtenu sous forme d'une huile jaune avec un rendement de 90 %. Les caractérisations RMN ¹H, ¹³C et IR confirment la structure du ligand L-3.
¹H NMR (300 MHz, CD₂Cl₂) : δ = 8.25 (s, 1H, *H₆*), 7.03 (d, *J* = 0.9 Hz, 1H, *H₃*), 6.93 (s, 1H, *H₂*), 3.95 (s, 3H, *H₁₁*), 3.63 (td, *J* = 6.7, 1.3 Hz, 2H, *H₈*), 2.72 (t, *J* = 6.8 Hz, 2H, *H₉*), 2.55 (q, *J* = 7.1 Hz, 4H, *H_{12.} H₁₄*), 1.00 (t, *J* = 7.1 Hz, 6H, *H₁₃, H₁₅*)*.*
¹³C NMR (75 MHz, CD₂Cl₂) : δ = 154.16 (*C₆*), 143.83 (*C₅*), 129.23 (*C₃*), 124.99 (*C₂*), 60.70 (*C₈*), 54.04 (*C₉*), 47.84 (*C₁₂*, *C₁₄*), 35.44 (*C₁₁*), 12.36 (*C₁₃*, *C₁₅*).
FT-IR : 2967w, 1650s, 1475m, 1437s, 1369w, 1287m, 1203w, 1148s, 1068w, 919w, 749m, 708m, 691m, 629s, 531s.

### Exemple 6

### Préparation du complexe Ni-3

Pour la synthèse du complexe, on opère comme dans l'Exemple 2 à ceci près que le ligand utiliser pour complexer le nickel est le ligand L-3. Le complexe Ni-3 est obtenu sous forme d'un solide orange avec un rendement de 89 %.

### Exemple 7

### Préparation du ligand L-4

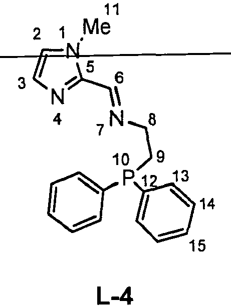

Pour la synthèse du ligand, on opère comme dans l'Exemple 1 à ceci près que l'amine utilisée est la 2-(diphénylphosphino)éthylamine. Le ligand L-4 est obtenu sous forme d'une huile jaune avec un rendement de 95 %. Les caractérisations RMN ¹H, ¹³C, ³¹P et IR confirment la structure du ligand L-4.
¹H NMR (300 MHz, CD₂Cl₂) : δ = 8.24 (s, 1H, *H₆),* 7.49-7.31 (m, 10H, *H₁₄, H₁₅*, *H₁₆*), 7.04 (d, *J* = 1.0 Hz, 1H, *H₃*), 6.92 (s, 1H, *H₂*), 3.85 (s, 3H, *H₁₁*), 3.77-3.66 (m, 2H, *H₈*), 2.48-2.43 (m, 2H, *H₉*).
³¹P NMR(121 MHz, CD₂Cl₂) : δ = -19.09 (s, 1P, *P₁₀*).
¹³C NMR (75 MHz, CD₂Cl₂) : δ = 153.95 (*C₆*), 143.55 (*C₅*), 139.23 (*C₁₂*), 133.23 (*C₁₃*), 129.35 (*C₃*), 128.86 (*C₁₄*), 128.77 (*C₁₅*), 58.95 (*C₈*), 35.47 (*C₁₁*), 30.22 (*C₉*).
FT-IR : 3051w, 1650s, 1518w, 1478m, 1434s, 1287w, 739m, 697m, 631m, 534s.

### Exemple 8

### Préparation du complexe Ni-4

Pour la synthèse du complexe, on opère comme dans l'Exemple 2 à ceci près que le ligand utiliser pour complexer le nickel est le ligand L-4. Le complexe Ni-4 est obtenu sous forme d'un solide orange avec un rendement de 80 %.

### Exemple 9

### Préparation du ligand L-5

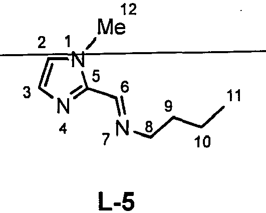

Pour la synthèse du ligand, on opère comme dans l'Exemple 1 à ceci près que l'amine utilisée est la butylamine. Le ligand L-5 est obtenu sous forme d'une huile jaune avec un rendement de 95 %. Les caractérisations RMN ¹H, ¹³C et IR confirment la structure du ligand L-5.
¹H NMR (300 MHz, CD₂Cl₂) : δ = 8.36 (d, *J* = 6.2 Hz, 1H, *H₆*), 7.12 (s, 1H, *H₃*), 7.01 (s, 1H, *H₂*), 4.04 (s, 3H, *H₁₂*), 3.65 (td, *J* = 6.8, 1.2 Hz, 2H, *H₈*), 1.73 (dq, *J* = 12.2, 6.9 Hz, 2H, *H₉*), 1.58-1.41 (m, 2H, *H₁₀*), 1.03 (t, *J* = 7.3 Hz, 3H, *H₁₁*).
¹³C NMR (75 MHz, CD₂Cl₂) : δ = 153.31 (*C₆*), 143.77 (*C₅*), 129.11 (*C₃*), 124.92 (*C₂*), 61.93 (*C₈*), 35.41 (*C₁₂*), 33.52 (*C₉*), 20.76 (*C₁₀*), 14.00 (*C₁₁*).
FT-IR : 3107w, 2930m, 1650s, 1476m, 1437s, 1366w, 1287w, 1149w, 1026w, 919w, 859w, 748m, 628s, 528s.

### Exemple 10

### Préparation du complexe Ni-5

Pour la synthèse du complexe, on opère comme dans l'Exemple 2 à ceci près que le ligand utiliser pour complexer le nickel est le ligand L-5. Le complexe Ni-5 est obtenu sous forme d'un solide bleu claire avec un rendement de 95 %.

### Exemple 11 :

### Préparation du ligand L-6

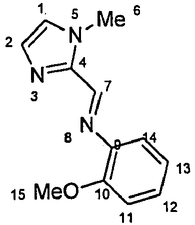

Pour la synthèse du ligand, on opère comme dans l'Exemple 1 à ceci près que l'amine utilisée est la ortho-anisidine. Le ligand L-6 est obtenu sous forme d'une huile orange avec un rendement de 95 %. Les caractérisations RMN ¹H, ¹³C et IR confirment la structure du ligand L-6.
¹H NMR (75 MHz, Acetone) : δ 8.47 (s, 1H, *H₇*), 7.34-6.90 (m, 6H, *H₁-H₂*, *H₁₁*-*H₁₄*), 4.14 (s, 3H, *H₁₅*), 3.85 (s, 3H, *H₆*).
¹³C NMR (75 MHz, Acetone) : δ 153.29 (*C₇*), 144.39 (*C₁₀*), 142.02 (*C₄*), 130.52 (*C₂*), 127.64 (*Cₐᵣₒₘ*), 126.78 (*Cₐᵣₒₘ*), 124.64 (*C₁*), 121.86 (*Cₐᵣₒₘ*), 121.37 (*Cₐᵣₒₘ*), 113.07 (*Cₐᵣₒₘ*), 56.23 (*C₁₅*), 35.84 (*C₆*).
FT-IR : 2951w, 2835w, 1685w, 1626s, 1585m, 1514m, 1430s, 1366w, 1288m, 1243s, 1149w, 1115m, 1048w, 1025m, 965w, 869m, 745s, 631m, 536s.

### Exemple 12

### Préparation du complexe Ni-6

Pour la synthèse du complexe, on opère comme dans l'Exemple 2 à ceci près que le ligand utiliser pour complexer le nickel est le ligand L-6. Le complexe Ni-6 est obtenu sous forme d'un solide vert claire avec un rendement de 95 %.

### Exemple 13

### Oligomérisation de l'éthylène

Le complexe de nickel (Ni-1 - Ni-6) est mis en solution dans le solvant de réaction puis est introduit dans le réacteur sous atmosphère d'éthylène. L'agent activateur est ensuite ajouté dans le réacteur. La température de la réaction est fixée à la température de test puis la pression ajustée à la pression de test (voir Tableau 1).
Après un temps t de réaction, l'introduction d'éthylène est stoppée. Le réacteur est refroidi et dégazé, puis les produits sont analysés par chromatographie en phase gaz.
La masse des produits formés et la distribution en oligomères sont données dans le Tableau 1.

**Tableau 1 : Oligomérisation de l'éthylène par les complexes Ni-1 et Ni-6.^{a}**

| **Entrée** | **Complexe** | **t (min)** | **m (g)^{d}** | **Distribution en oligomères^{e}** | |
|---|---|---|---|---|---|
| | | | | **C₄** | **C₆** |
| 1^{a} | **Ni-1** | 55 | 31,0 | 69 | 31 |
| 2^{a} | **Ni-2** | 60 | 9,3 | 78 | 22 |
| 3^{a} | **Ni-3** | 60 | 3,6 | 78 | 22 |
| 4^{a} | **Ni-4** | 60 | 17,6 | 82 | 17 |
| 5^{a} | **Ni-5** | 60 | 21,2 | 66 | 34 |
| 6^{b} | **Ni-1** | 52 | 30,1 | 71 | 29 |
| 7^{b} | **Ni-2** | 58 | 29,5 | 70 | 30 |
| 8^{b} | **Ni-3** | 58 | 29,7 | 71 | 29 |
| 10^{b} | **Ni-5** | 58 | 30,1 | 70 | 30 |
| 11^{c} | **Ni-1** | 20 | 66,8 | 84 | 16 |
| 12^{c} | **Ni-2** | 73 | 62,0 | 87 | 13 |
| 13^{c} | **Ni-6** | 20 | 64,5 | 87 | 13 |

| | | | | | |
|---|---|---|---|---|---|
| ^{a} Heptane (25 mL), Ni (20 µmol), pression d'éthylène 0,5 MPa, 45°C, activateur : EtAlCl₂ (Al/Ni=15). ^{b} Toluène (25 mL), Ni (20 µmol), pression d'éthylène 0,5 MPa, 45°C, activateur : EtAlCl₂ (Al/Ni=15). ^{c} Heptane (100 mL), Ni (10 µmol), pression d'éthylène 3 MPa, 45°C, activateur : EtAlCl₂ (Al/Ni=15). ^{d} correspond à la masse d'oligomères produits au cours du test. ^{e}% pds, déterminée par GC. | | | | | |

Les exemples ci-dessus démontrent que l'utilisation d'une nouvelle composition catalytique selon l'invention permet d'obtenir une sélectivité très élevée pour la dimérisation de l'éthylène.

## Revendications

1. Composition catalytique pour l'oligomérisation des oléfines comportant de 2 à 10 atomes de carbone, ladite composition catalytique comprenant au moins un complexe de nickel, ledit complexe étant obtenu à partir d'un mélange comprenant :
- au moins un précurseur de nickel A, avec
- au moins un ligand imino-imidazole B de formule (I) 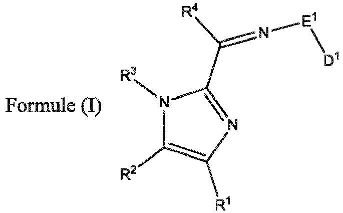
dans laquelle R¹, R², R³ et R⁴ sont identiques ou différents et choisis parmi l'atome d'hydrogène, les groupements alkyles linéaires ou ramifiés, les groupements aryles, aralkyles ou alkaryles comportant 1 à 12 atomes de carbone et contenant ou non des hétéroéléments, R¹ et R² ne formant pas de cycle aromatique;
E¹ est un groupement choisi parmi les groupes -CH₂-, -CH₂CH₂-, -CH₂CH₂CH₂-,-CH₂CH₂CH₂CH₂-, 1,2-phenylène, *trans*-1,2-cyclopentane, *trans*-1,2-cyclohexane, 2,3-butane, 1,1'-biphényle, 1,1'-binaphthyle et -Si(Me)₂- ;
D¹ est choisi parmi un atome d'hydrogène, un éther de formule -OR⁵, un thioéther de formule -SR⁶, une amine de formule -N(R⁷)₂ ou une phosphine de formule -P(R⁸)₂ où R⁵, R⁶, R⁷ et R⁸ sont des groupements méthyles, éthyles ou phényle.

2. Composition catalytique selon la revendication 1 dans laquelle R¹, R², R³ et R⁴ sont choisis parmi les groupements hydrogène, méthyle, éthyle, isopropyle, *iso*-butyle, *tert*-butyle, cyclohexyle, phényle et benzyle.

3. Composition catalytique selon l'une des revendications 1 ou 2 dans laquelle R¹, R² et R⁴ sont des atomes d'hydrogène.

4. Composition catalytique selon l'une des revendications 1 à 3 dans laquelle R³ est un groupement méthyle.

5. Composition catalytique selon la revendication 1 dans laquelle E¹ est le groupement divalent -CH₂CH₂- ou le groupement divalent 1,2-phenylène.

6. Composition catalytique selon l'une des revendications 1 à 5 dans laquelle ledit précurseur de nickel A utilisé dans la composition catalytique selon l'invention est choisi parmi le chlorure de nickel(II), le chlorure de nickel(II)(dimétoxyéthane), le bromure de nickel(II), le bromure de nickel(II)(dimétoxyéthane), le fluorure de nickel(II), l'iodure de nickel(II), le sulfate de nickel(II), le carbonate de nickel(II), le dimethylglyoxime de nickel(II), l'hydroxyde de nickel(II), l'hydroxyacétate de nickel(II), l'oxalate de nickel(II), les carboxylates de nickel(II), le 2-éthylhexanoate, les phénates de nickel, l'acétate de nickel(II), le trifluoroacétate de nickel(II), le triflate de nickel(II), l'acétylacétonate de nickel(II), l'hexafluoroacétylacétonate de nickel(II), le nickel bis(cycloocta-1,5-diène), le nickel bis(cycloocta-1,3-diène), le nickel bis(cyclooctatétraène), le nickel bis(cycloocta-1,3,7-triène), le bis(o-tolylphosphito)nickel(éthylène), le nickel tétrakis (triphénylphosphite), le nickel bis(éthylène), le chlorure de π-allylnickel(II), le bromure de π-allylnickel(II), le dimère du chlorure de methallylnickel(II), l'hexafluorophosphate de η³-allylnickel(II), l'hexafluorophosphate de η³-methallylnickel(II) et le 1,5-cyclooctadiényle de nickel(II), sous leur forme hydraté ou non, pris seul ou en mélange, complexés ou non à des bases de Lewis.

7. Composition catalytique selon l'une des revendications 1 à 6 dans laquelle la composition catalytique contient un composé C, appelé agent activateur, choisi dans le groupe formé par les composés tris(hydrocarbyl)aluminium, les composés chlorés ou bromés d'hydrocarbylaluminium, les aluminoxanes, les composés organiques susceptibles de donner un proton, les composés organo-borés, pris seuls ou en mélange.

8. Composition catalytique selon l'une des revendications 1 à 7 dans laquelle ledit complexe de nickel de la composition catalytique peut avantageusement être préparé en présence d'un solvant ou pas.

9. Procédé d'oligomérisation des oléfines comportant de 2 à 10 atomes de carbone, comprenant la mise en contact desdites oléfines avec ladite composition catalytique selon l'une des revendications 1 à 8.

10. Procédé selon la revendication 9 dans lequel les oléfines sont choisies parmi l'éthylène, le propylène, les n-butènes et les n-pentènes, seuls ou en mélange, purs ou dilués.

11. Procédé selon la revendication 10 dans lequel ledit procédé est un procédé d'oligomérisation de l'éthylène.

12. Procédé selon la revendication 11 dans lequel ledit procédé est un procédé de dimérisation de l'éthylène.

## Patentansprüche

1. Katalytische Zusammensetzung zur Oligomerisierung von Olefinen mit 2 bis 10 Kohlenstoffatomen, wobei die katalytische Zusammensetzung mindestens einen Nickel-Komplex umfasst, wobei der Komplex aus einer Mischung erhalten wird, umfassend:
- mindestens einen Nickel-Vorläufer A mit
- mindestens einem Imino-Imidazol-Liganden B mit der Formel (I): 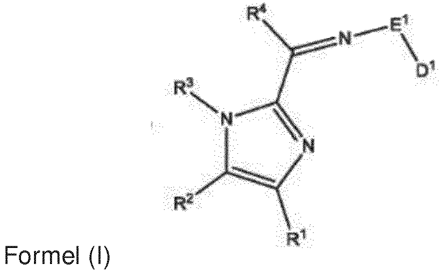
wobei R¹, R², R³ und R⁴ identisch oder verschieden sind und ausgewählt sind aus einem Wasserstoffatom, linearen oder verzweigten Alkylgruppen, Arylgruppen, Aralkyl- oder Alkarylgruppen mit 1 bis 12 Kohlenstoffatomen und enthaltend Heteroelemente oder nicht, wobei R¹ und R² keinen aromatischen Ring bilden;
E¹ eine Gruppe ist, ausgewählt aus den Gruppen -CH₂-, -CH₂CH₂-, -CH₂CH₂CH₂-, -CH₂CH₂CH₂CH₂-, 1,2-Phenylen, trans-1,2-Cyclopentan, trans-1,2-Cyclohexan, 2,3-Butan, 1,1'-Biphenyl, 1,1'-Binaphthyl und -Si(Me)₂-;
D¹ ausgewählt ist aus einem Wasserstoffatom, einem Ether mit der Formel -OR⁵, einem Thioether mit der Formel -SR⁶, einem Amin mit der Formel -N(R⁷)₂ oder einem Phosphin mit der Formel -P(R⁸)₂, wobei R⁵, R⁶, R⁷ und R⁸ Methyl-, Ethyl- oder Phenylgruppen sind.

2. Katalytische Zusammensetzung nach Anspruch 1, wobei R¹, R², R³ und R⁴ ausgewählt sind aus Wasserstoff-, Methyl-, Ethyl-, Isopropyl-, Isobutyl-, tert-Butyl-, Cyclohexyl-, Phenyl- und Benzylgruppen.

3. Katalytische Zusammensetzung nach einem der Ansprüche 1 oder 2, wobei R¹, R² und R⁴ Wasserstoffatome sind.

4. Katalytische Zusammensetzung nach einem der Ansprüche 1 bis 3, wobei R³ eine Methylgruppe ist.

5. Katalytische Zusammensetzung nach Anspruch 1, wobei E¹ eine zweiwertige Gruppe -CH₂CH₂- oder eine zweiwertige 1,2-Phenylengruppe ist.

6. Katalytische Zusammensetzung nach einem der Ansprüche 1 bis 5, wobei der Nickel-Vorläufer A, der in der katalytischen Zusammensetzung gemäß der Erfindung verwendet wird, ausgewählt ist aus Nickel(II)-chlorid, Nickel(II)-(dimethoxyethan)-chlorid, Nickel(II)-bromid, Nickel(II)-(dimethoxyethan)-bromid, Nickel(II)-fluorid, Nickel(II)-iodid, Nickel(II)-sulfat, Nickel(II)-carbonat, Nickel(II)-dimethylglyoxim, Nickel(II)-hydroxid, Nickel(II)-hydroxyacetat, Nickel(II)-oxalat, Nickel(II)-carboxylaten, 2-Ethylhexanoat, Nickelphenaten, Nickel(II)-acetat, Nickel(II)-trifluoracetat, Nickel(II)-triflat, Nickel(II)-acetylacetonat, Nickel(II)-hexafluoracetylacetonat, Nickel-bis(cycloocta-1,5-dien), Nickel-bis(cycloocta-1,3-dien), Nickel-bis(cyclooctatetraen), Nickel-bis(cycloocta-1,3,7-trien), bis(o-Tolylphosphito)-nickel-(ethylen), Nickeltetrakis-(triphenylphosphit), Nickel-bis(ethylen), Π-Allylnickel(II)-chlorid, Π-Allylnickel(II)-bromid, dem Dimer von Methallylnickel(II)-chlorid, η³-Allylnickel(II)-hexafluorphosphat, η³-Methallylnickel(II)-hexafluorphosphat und Nickel(II)-1,5-cyclooctadienyl, in ihrer hydratisierten Form oder nicht, allein oder in Mischung, komplexiert mit Lewis-Basen oder nicht.

7. Katalytische Zusammensetzung nach einem der Ansprüche 1 bis 6, wobei die katalytische Zusammensetzung eine Verbindung C enthält, die als Aktivatormittel bezeichnet wird, ausgewählt aus der Gruppe gebildet von Tris-(hydrocarbyl)-aluminium, chlorierten oder bromierten Verbindungen von Hydrocarbylaluminium, Aluminoxanen, organischen Verbindungen, die ein Proton spenden können, organo-bortierten Verbindungen, allein oder in Mischung.

8. Katalytische Zusammensetzung nach einem der Ansprüche 1 bis 7, wobei der Nickel-Komplex der katalytischen Zusammensetzung vorteilhaft in Anwesenheit eines Lösungsmittels oder nicht hergestellt werden kann.

9. Verfahren zur Oligomerisierung von Olefinen mit 2 bis 10 Kohlenstoffatomen, umfassend das Inkontaktbringen der Olefine mit der katalytischen Zusammensetzung nach einem der Ansprüche 1 bis 8.

10. Verfahren nach Anspruch 9, wobei die Olefine ausgewählt werden aus Ethylen, Propylen, n-Butenen und n-Pentenen, allein oder in Mischung, rein oder verdünnt.

11. Verfahren nach Anspruch 10, wobei das Verfahren ein Verfahren zur Oligomerisierung von Ethylen ist.

12. Verfahren nach Anspruch 11, wobei das Verfahren ein Verfahren zur Dimerisierung von Ethylen ist.

## Claims

1. A catalytic composition for the oligomerization of olefins having from 2 to 10 carbon atoms, the catalytic composition comprising at least one nickel complex, the complex being obtained from a mixture comprising:
- at least one nickel precursor A with
- at least one imino-imidazole ligand B having the formula (I): 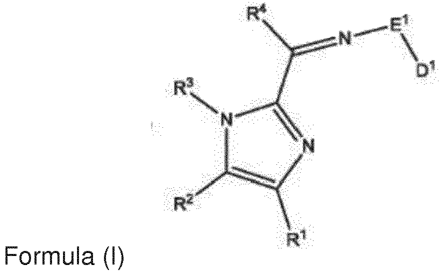
wherein R¹, R², R³ and R⁴ are identical or different and are selected from hydrogen, linear or branched alkyl groups, aryl groups, aralkyl or alkaryl groups of 1 to 12 carbon atoms and containing hetero elements or not, wherein R 1 and R 2 do not form an aromatic ring;
E1 is a group selected from CH₂, CH₂CH₂, CH₂CH₂CH₂, CH₂CH₂CH₂CH₂, 1,2-phenylene, trans-1,2-cyclopentane, trans-1,2-cyclohexane, 2,3-butane, 1,1'-biphenyl 1,1'-binaphthyl and Si(Me)₂;
D1 is selected from a hydrogen atom, an ether of the formula -OR⁵, a thioether of the formula SR⁶, an amine of the formula N(R⁷)₂ or a phosphine of the formula P(R⁸)₂, wherein R⁵, R⁶, R⁷ and R⁸ are methyl, ethyl or phenyl groups.

2. A catalytic composition according to claim 1, wherein R1, R2, R3 and R4 are selected from hydrogen, methyl, ethyl, isopropyl, isobutyl, tert-butyl, cyclohexyl, phenyl and benzyl groups.

3. A catalytic composition according to any one of claims 1 or 2, wherein R¹, R² and R⁴ are hydrogen atoms.

4. A catalytic composition according to any one of claims 1 to 3, wherein R 3 is a methyl group.

5. A catalytic composition according to claim 1, wherein E1 is a divalent group CH₂CH₂ or a divalent 1,2-phenylene group.

6. A catalytic composition according to any one of claims 1 to 5, wherein the nickel precursor A used in the catalytic composition according to the invention is selected from from nickel(II) chloride, nickel(II) chloride (dimethoxyethane), nickel(II) bromide, nickel(II) bromide (dimethoxyethane), nickel(II) fluoride, nickel(II) iodide, nickel(II) sulphate, nickel(II) carbonate, nickel(II) dimethylglyoxime, nickel(II) hydroxide, nickel(II) hydroxyacetate, nickel(II) oxalate, nickel(II) carboxylates such as for example 2-ethylhexanoate, nickel phenates, nickel(II) acetate, nickel(II) trifluoroacetate, nickel(II) triflate, nickel(II) acetylacetonate, nickel(II) hexafluoroacetylacetonate, nickel bis(cycloocta-1,5-diene), nickel bis(cycloocta-1,3-diene), nickel bis(cyclooctatetraene), nickel bis(cycloocta-1,3,7-triene), bis(o-tolylphosphito)nickel(ethylene), nickel tetrakis(triphenylphosphite), nickel bis(ethylene), π-allylnickel(II) chloride, π-allylnickel(II) bromide, methallylnickel(II) chloride dimer, η³-allylnickel(II) hexafluorophosphate, η³-methallylnickel(II) hexafluorophosphate and nickel(II) 1,5-cyclooctadienyl, in their hydrated or unhydrated form, used alone or in a mixture, complexed or not with Lewis bases.

7. A catalytic composition according to any one of claims 1 to 6 wherein the catalytic composition comprises a compound C referred to as an activator selected from the group formed by tris(hydrocarbyl)aluminium compounds, chlorinated or brominated hydrocarbylaluminium compounds, aluminoxanes, organic compounds capable of acting as proton donor, organoboron compounds, used alone or in a mixture.

8. A catalytic composition according to any one of claims 1 to 7, wherein the nickel complex of the catalytic composition can be advantageously produced in the presence of a solvent or not.

9. A process for the oligomerization of olefins having 2 to 10 carbon atoms, comprising contacting the olefins with the catalytic composition according to any one of claims 1 to 8.

10. A process according to claim 9, wherein the olefins are chosen from ethylene, propylene, n-butenes and n-pentenes, alone or in admixture, pure or diluted.

11. A process according to claim 10, wherein the process is a process for the oligomerization of ethylene.

12. A process according to claim 11, wherein the process is a process for dimerizing ethylene.
